# EUROPÄISCHE PATENTANMELDUNG

(11) **EP 0 895 751 A1**
(43) Veröffentlichungstag der Anmeldung: **10.02.1999**
(21) Anmeldenummer: 98113841.5
(22) Anmeldetag: 23.07.1998
(51) Int. Cl.: A61B 5/14

(54) **Blutentnahmevorrichtung**

(30) Priorität: 23.07.1997 DE 19731742
(71) Anmelder: pvb medizintechnik gmbh & co. kg, 85613 Kirchseeon/Eglharting (DE)
(72) Erfinder: Beck, Bernd, 72414 (DE)
(74) Vertreter: von Bülow, Tam, Dr.

(57) **Zusammenfassung**

Die Blutentnahmevorrichtung (1) enthält einen Entnahmeport (7) und zwei Hähne (5, 6), die jeweils mit dem Entnahmeport (7) verbunden sind. Der Entnahmeport (7) weist einen Hohlraum (104) auf, in den zwei Kanäle (107, 108) einmünden. Der Hohlraum (104) hat eine Öffnung, die durch einen Verschlußstopfen (46) verschlossen ist. Der Verschlußstopfen (46) kann zum Entnehmen von Blut aus dem Hohlraum (104) mit einer Nadel eines Blutentnahmegeräts durchstochen werden. Der Entnahmeport (7) und die beiden Hähne (5, 6) sind raumfest zueinander angeordnet. Ferner ist ein bewegliches Abdeckorgan (49) vorgesehen, das in einer seiner möglichen Stellungen den Verschluß (46) des Entnahmeports (7) abdeckt und ihn in einer anderen Stellung freigibt. Die Hähne (5, 6) und das Abdeckorgan (49) sind durch ein gemeinsames Betätigungsorgan (3) der Blutentnahmevorrichtung (1) mechanisch miteinander gekoppelt. Durch die Stellung des Betätigungsorganes (3) sind die Stellungen beider Hähne (5, 6) und des Abdeckorganes (49) festgelegt. Durch Betätigen des Betätigungsorganes (3) können konstruktiv vorgebbare Stellungskombinationen der beiden Hähne (5, 6) und des Abdeckorganes (49) der Blutentnahmevorrichtung (1) eingestellt werden.

## Beschreibung

Die Erfindung bezieht sich auf eine Blutentnahmevorrichtung mit einem Entnahmeport und zwei mit dem Entnahmeport verbundenen Hähnen gemäß dem Oberbegriff des Patentanspruches 1.

Eine derartige Blutentnahmevorrichtung ist aus einem Firmenprospekt der Firma pvb medizintechnik gmbh mit dem Titel "Haemoguard, the closest arterial blood sampling system for use during invasive blood pressure monitoring" (November 1993) bekannt, jedoch mit der Maßgabe, daß dort nur ein Hahn verwendet wird; in der Praxis werden jedoch in manchen Fällen auch zwei Hähne verwendet.

Der in dieser Druckschrift gezeigte Absperrhahn ist über eine flexible Leitung mit einer Blutentnahmestelle verbunden, die im folgenden als "Entnahmeport" bezeichnet wird. Dieser Entnahmeport ist mittels eines flexiblen Schlauches in einem Abstand zum Hahn angeordnet, ohne daß eine bestimmte Position relativ zum Hahn festgelegt ist.

Der Entnahmeport hat einen Hohlraum, in den von gegenüberliegenden Seiten je ein Kanal einmündet. Diese Kanäle sind mit je einer Leitung zum Leiten von Blut und/oder einer Infusionsflüssigkeit verbunden. Ferner weist der Hohlraum des Entnahmeports eine Öffnung auf, die durch einen Verschlußstopfen flüssigkeitsdicht verschlossen ist. Der Verschlußstopfen ist aus einem Elastomerwerkstoff hergestellt und kann mit einer Blutentnahmenadel durchstochen werden, um eine im Hohlraum des Entnahmeports enthaltene Flüssigkeit, wie z.B. Blut, zu entnehmen.

Der Entnahmeport ist üblicherweise in einem Fluidsystem angeordnet, das Einrichtungen zur Infusion von Medikamenten und/oder zur arteriellen oder intravenösen Blutdruckmessung aufweist. Beispielsweise ist ein solches System wie folgt aufgebaut:

Ausgehend von einer in ein Blutgefäß des Patienten eingeführten Nadel führt ein Schlauch über einen ersten Absperrhahn zum Entnahmeport, von dort über einen zweiten Absperrhahn zu einem Reservoir und von dort über einen dritten Absperrhahn zu einem Druckmeßfühler und von dort zu einem Behälter für Spülflüssigkeit, wie z.B. physiologischer Kochsalzlösung, deren Zweck es ist, das Leitungssystem freizuhalten und Blutkoagulationen im Leitungssystem zu verhindern. Während der Blutdruckmessung sind alle genannten drei Absperrhähne geöffnet. Am Entnahmeport befindet sich je nach Schlauchlänge in diesem Zustand reine Spülflüssigkeit oder mit Spülflüssigkeit verdünntes Blut. Soll nun für Analysezwecke am Entnahmeport Blut entnommen werden, so muß vorher dafür Sorge getragen werden, daß dort reines, unverdünntes Blut vorhanden ist. Hierzu wird zunächst der dritte Hahn abgesperrt, um ein Nachspeisen von Spülflüssigkeit zu unterbinden. Die ersten und zweiten Absperrhähne sind noch geöffnet. Nun wird das Reservoir gefüllt, so daß Spülflüssigkeit und verdünntes Blut aus dem Leitungssystem soweit zurückgezogen werden, daß am Entnahmeport nur noch reines Blut vorhanden ist. Anschließend wird der zweite Absperrhahn geschlossen und am Entnahmeport kann reines Blut entnommen werden. Nach der Entnahme wird der zweite Absperrhahn wieder geöffnet und der Inhalt des Reservoirs wird in das System zurück zum Patienten gedrückt. Abschließend wird dann auch der dritte Hahn wieder geöffnet.

Die korrekte Bedienung des Systems erfordert eine gründliche Schulung des medizinischen Personals und auch eine hohe Aufmerksamkeit des Personals, die in Streßsituationen nicht immer gegeben ist. So kann es beispielsweise vorkommen, daß am Entnahmeport noch verdünntes Blut vorliegt und somit eine für Analysezwecke unbrauchbare Blutprobe entnommen wird. Eine andere Fehlbedienung kann darin liegen, daß während des Füllens des Reservoirs der zweite und der dritte Hahn geschlossen sind. Dadurch wird im Reservoir ein Unterdruck erzeugt, der beim anschließenden Öffnen des zweiten Hahns zu einer Gasblasenbildung führen kann. Gleiches kann theoretisch auftreten, wenn der erste und der zweite Hahn während der Blutentnahme am Entnahmeport geschlossen sind.

Aufgabe der Erfindung ist es daher, eine Blutentnahmevorrichtung zu schaffen, die sicherstellt, daß eine Blutentnahme am Entnahmeport nur bei ordnungsgemäßer Stellung beider mit dem Entnahmeport verbundener Hähne möglich ist.

Diese Aufgabe wird durch die im Patentanspruche 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, die beiden Hähne beidseitig des Entnahmeports und ein Abdeckorgan für diesen derart zu koppeln, daß der Entnahmeport nur dann für eine Blutentnahme zugänglich ist, wenn beide Hähne in ihrer hierfür erforderlichen Stellung sind.

Dies hat den großen Vorteil, daß eine Blutentnahme aus dem Entnahmeport nur in einer oder mehreren bestimmten, konstruktiv vorgebbaren Stellungskombinationen der beiden Hähne möglich und ansonsten durch das Abdeckorgan blockiert ist. Beispielsweise ist es sinnvoll, die Blutentnahmevorrichtung so zu konzipieren, daß ein Zugang zum Verschlußstopfen des Entnahmeports nur dann möglich ist, wenn der "vor" dem Entnahmeport angeordnete Hahn offen und der nach dem Entnahmeport angeordnete Hahn geschlossen ist. Bei allen anderen Stellungskombinationen der Hähne ist dagegen der Zugang zum Verschlußstopfen blockiert. Im Gegensatz zum Stand der Technik ist es somit bei nicht ordnungsgemäßer Stellung der Hähne unmöglich, eine Blutennahme durchzuführen. Eine Fehlbedienung der Blutenentnahmevorrichtung ist damit ausgeschlossen.

Ein weiterer Vorteil der Erfindung besteht darin, daß die Blutentnahmevorrichtung so aufgebaut ist, daß die bei einer ordnungsgemäßen Blutentnahme nacheinander einzustellende Stellungskombinationen der Hähne sequentiell durchlaufen werden. Eine mit der Blutentnahme beauftragte Krankenschwester braucht sich somit nicht unbedingt jede der einzustellenden Hahnstellungskombinationen und deren Einstellungsreihenfolge zu merken, sondern kann diese programmartig durch einfaches Betätigen des Betätigungsorgans der Blutentnahmevorrichtung, wie z.B. durch Drehen eines Bedienknopfes in einer Drehrichtung durchlaufen.

Realisierbar ist eine derartige Blutentnahmevorrichtung dadurch, daß ein bewegliches Abdeckorgan vorgesehen ist, welches in denjenigen Stellungskombinationen der Hähne den Verschluß des Entnahmeports abdeckt, in denen keine Blutentnahme erfolgen darf und welches in einer oder mehreren anderen Stellungskombinationen, in denen eine Blutentnahme erfolgen kann, den Entnahmeport zur Blutentnahme freigibt. Um dies rein mechanisch zu erreichen, sind die beiden Hähne und der Entnahmeport raumfest zueinander angeordnet. Ferner weist die Blutentnahmevorrichtung ein Betätigungsorgan auf, über das die an den Entnahmeport angeschlossenen Hähne und das Abdeckorgan, das den Verschluß des Entnahmeports in bestimmten Stellungen freigibt bzw. abdeckt, mechanisch gekoppelt sind. Durch Betätigen des Betätigungsorganes ist es möglich, die Stellungskombination der beiden Hähne zu ändern und das Abdeckorgan dementsprechend zu bewegen, so daß der Verschluß freigegeben bzw. abgedeckt ist.

Wird, ausgehend von einer momentan eingestellten Stellungskombination der Hähne, die bei der Blutentnahme nächstfolgende Stellungskombination durch Betätigen des Betätigungsorganes angefahren, so werden je nach momentaner Stellungskombination entweder beide Hähne oder auch nur ein Hahn verstellt; gleichzeitig wird das Abdeckorgan entweder bewegt, so daß es in Abhängigkeit von der momentanen Stellungskombination der Hähne den Verschluß des Entnahmeports freigibt bzw. abdeckt, oder es verbleibt in der Stellung, die es innehat. Somit ist allein durch Betätigen des einen Betätigungsorgans der Blutentnahmevorrichtung eine Umsetzung des für eine ordnungsgemäße Blutentnahme erforderlichen Bewegungsablaufes der Hähne und des Abdeckorganes möglich. Die Stellungskombinationen der Hähne und die zu jeder Stellungskombination gehörende Stellung des Abdeckorganes sind dabei durch die Stellung des gemeinsamen Betätigungsorganes festgelegt.

Nach einer Weiterbildung der Erfindung erfolgt die mechanische Kopplung zwischen dem Betätigungsorgan und den Hähnen durch eine Verzahnung. Hierfür sind an beiden Hähnen und am Betätigungsorgan Zähne vorgesehen, die in Eingriff gebracht werden können. Da zahlreiche medizinische Geräte, insbesondere medizinische Hähnchen, im Spritzgußverfahren aus Kunststoff hergestellt werden, ist es möglich, beim Spritzen des Hahnes die Zähne im selben Prozeßgang mit anzuspritzen. Somit ist die mechanische Kopplung des Betätigungsorgans mit den beiden Hähnen durch eine Verzahnung mit relativ geringen Herstellkosten verbunden. Ein weiterer wesentlicher Vorteil einer derartigen Verzahnung besteht darin, daß die "Offen"- bzw. "Geschlossen"-Positionen der Hähne auch bei mehrmaliger Betätigung des Betätigungsorgans genau festgelegt bleiben.

Nach einer Weiterbildung der Erfindung sind die Zähne am Betätigungsorgan in azimutaler Richtung in einzelnen Abschnitten, d.h. mit Lücken zwischen einzelnen Zahnabschnitten angeordnet. Somit gibt es einerseits Stellungen des Betätigungsorganes, in denen Zähne des Betätigungsorganes mit den Zähnen eines oder beider Hähne im Eingriff sind und andererseits gibt es auch Stellungen des Betätigungsorganes, in denen kein Eingriff stattfindet. Je nach momentaner Stellung des Betätigungsorganes ist es also möglich, eine Bewegung des Betätigungsorganes in eine Bewegung beider Hähne, nur eines Hahnes oder gar keines Hahnes umzusetzen. Auf diese Weise können nacheinander problemlos einzelne Stellungskombinationen durch Betätigen des Betätigungsorganes eingstellt werden. Beispielsweise ist es möglich, die Blutentnahmevorrichtung derart zu konzipieren, daß ausgehend von einer Stellung in der beide Hähne geschlossen sind, durch einen einzigen Betätigsschritt eine Stellungskombination herbeigeführt wird, in der einer der beiden Hähne oder auch beide Hähne offen sind. In Abhängigkeit von der Anzahl der Zahnabschnitte, ihrer Längen und der Längen der Lücken lassen sich somit konstruktiv unterschiedliche "Bewegungsprogramme" für die beiden Hähne festlegen.

Nach einer Weiterbildung der Erfindung liegen die Zähne des einen Hahnes gegenüber den Zähnen des anderen Hahnes in einer anderen axial versetzten Ebene. Ferner können auch einzelne Zähne bzw. Zahnabschnitte des Betätigungsorgans axial versetzt sein und/oder unterschiedliche Zahnbreite haben. Durch eine derartige Zähneanordnung ist es möglich, daß bestimmte Zähne des Betätigungsorganes entweder ausschließlich in die Zähne eines oder auch beider Hähne eingreifen. Ferner ist es auch möglich, am Betätigungsorgan Zahnabschnitte vorzusehen, deren Zähne so breit sind, daß sie in bestimmten Stellungen des Betätigungsorganes nacheinander oder auch gleichzeitig in Zähne eines jeden der beiden Hähne eingreifen. Durch eine derartige Gestaltung der Verzahnung des Betätigungsorganes ist möglich, insbesondere in Verbindung mit der erläuterten abschnittsweisen Zahnanordnung, für jeden Hahn eine "eigene", den Bewegungsablauf des Hahns bestimmte Verzahnung am Betätigungsorgan vorzusehen; durch eine derartige Verzahnung in "mehreren Zahnebenen" lassen sich im Vergleich zu einer Verzahnung in nur einer Ebene mehr und konstruktiv leichter zu überblickende Bewegungsabläufe realisieren.

Zusätzlich zu den versetzten und eventuell unterschiedlich breiten Zähnen des Betätigungsorgans können die einzelnen Zähne an den beiden Hähnen unterschiedlich breit gestaltet werden. Hierdurch ist es möglich, daß ein in einer bestimmten Ebene am Betätigungsorgan angeordneter Zahn nur bestimmte, d. h. verbreiterte Zähne des entsprechenden Hahns mitnimmt, wodurch sich die Anzahl realisierbarer Bewegungsabläufe zusätzlich erhöht.

Nach einer Weiterbildung der Erfindung ist an den Hähnen jeweils eine Außenverzahnung und am Betätigungsorgan eine Innenverzahnung vorgesehen. In diesem Fall sind die Hähne bzw. die Verzahnungen der Hähne von der Verzahnung des Betätigungsorganes umschlossen. Alternativ dazu ist es auch möglich, sowohl das Betätigungsorgan als auch die Hähne jeweils mit einer Außenverzahnung zu versehen. In diesem Fall sind die Hähne bzw. deren Verzahnungen außen an der Verzahnung des Betätigungsorganes, ähnlich den Planeten eines Planetengetriebes, angeordnet.

Für die Bewegung des Abdeckorganes durch das Betätigungsorgan ist nach einer Weiterbildung der Erfindung am Betätigungsorgan ein Mitnehmerelement und am Abdeckorgan ein mit dem Mitnehmerelement zusammenwirkendes Anschlagelement vorgesehen. In manchen Stellungsbereichen des Betätigungsorganes befindet sich das Mitnehmerelement in Positionen, in denen es das Anschlagelement berührt bzw. auslenkt. Anstatt nur eines Mitnehmerelements können am Betätigungsorgan auch mehrere Mitnehmerelemente angebracht sein, so daß bei einem vollständigen "Programmdurchlauf" der Blutentnahmevorrichtung das Abdeckorgan mehrmals geöffnet bzw. geschlossen wird.

Ferner ist ein Federelement vorgesehen, das mit dem Abdeckorgan zusammenwirkt. Bei einer durch das Mitnehmerelement des Betätigungsorgans bewirkten Auslenkung des Abdeckorgans aus einer seiner möglichen Stellungen in eine andere wirkt eine durch das Federelement aufgebrachte Federkraft der Bewegung entgegen. Ab einer konstruktiv vorgebbaren Maximalauslenkung des Abdeckorganes durch das Mitnehmerelement des Betätigungsorganes wird durch Weiterbewegen des Betätigungsorganes die "Kontaktierung" des Mitnehmerelementes mit dem Anschlag des Abdeckorganes unterbrochen; die Feder bewirkt nun eine Rückführung des Abdeckorganes in seine Ausgangsstellung, d.h. in die Stellung bevor das Mitnehmerelement des Betätigungsorganes den Anschlag berührte.

Vorzugsweise ist das Abdeckorgan als Abdeckarm ausgeführt und an einem der beiden Hähne gelagert, so daß er von einer "Abdeckstellung" in eine "Freigabestellung" und umgekehrt schwenkbar ist. Eine Lagerung an einem der beiden Hähne spart ein separates Lagerelement ein. Ferner läßt sich dadurch eine sehr kompakte Anordnung des Abdeckorganes in der Blutentnahmevorrichtung erreichen.

Alternativ zu dem beschriebenen Mechanismus, bei dem ein Mitnehmerelement mit einem Anschlagelement und einem Federelement zusammenwirkt, ist es nach einer Weiterbildung der Erfindung auch möglich, das Abdeckorgan als drehbare Abdeckscheibe auszuführen, die eine oder mehrere Durchgangsöffnungen aufweist. Die Abdeckscheibe ist relativ zum Entnahmeport derart angeordnet, daß in manchen ihrer möglichen Drehstellungen eine Durchgangsöffnung über dem Verschluß des Entnahmeports liegt und dieser somit zugänglich ist. In anderen Drehstellungen der Abdeckscheibe ist der Verschluß des Entnahmeports durch die Abdeckscheibe abgedeckt.

Für die "Steuerung" des Bewegungsablaufs der Abdeckscheibe kann z. B., ähnlich wie zur Steuerung der Hahnstellungen, eine kostengünstige Verzahnung verwendet werden. Beispielsweise kann am Betätigungsorgan eine separate "Verzahnungsebene" für das Abdeckorgan und am Abdeckorgan eine mit dieser Verzahnung zusammenwirkende Verzahnung vorgesehen werden.

Als weitere konstruktive Variante ist es auch möglich, die Abdeckscheibe derart zu gestalten und anzuordnen, daß sie neben der Abdeckfunktion auch die Funktion des Betätigungsorgans der Blutentnahmevorrichtung übernimmt. Für diesen Zweck kann die Abdeckscheibe beispielsweise relativ groß in Bezug auf die Blutentnahmevorrichtung ausgeführt sein und ein drehbares Außenteil der Blutentnahmevorrichtung bilden; hierdurch läßt sich das Bauteil, das ausschließlich die Betätigungsfunktion übernimmt, einsparen.

Vorzugsweise ist das Betätigungsorgan der Blutentnahmevorrichtung als deckelartiger Drehgriff ausgeführt, der beide Hähne und den Entnahmeport überdeckt und über dem Verschluß des Entnahmeports eine Öffnung aufweist. An der Öffnung kann eine Führungsfläche vorgesehen sein, die mit einem durch die Öffnung in Richtung zum Verschluß des Entnahmeports einzuführenden Blutentnahmegerätes zusammenwirkt. Ein derart gestalteter deckelartiger Drehgriff ist ein in Bezug auf die gesamte Blutentnahmevorrichtung relativ großes und somit leicht zu betätigendes Bauteil. Durch die Kombination des Drehgriffes mit einer mit einem Blutentnahmegerät zusammenwirkenden Führungsfläche ist es möglich, ein entsprechendes Blutentnahmegerät sicher und in ordnungsgemäßer Stellung in Bezug auf die Blutentnahmevorrichtung an den Entnahmeport anzusetzen. Durch eine derartige Führung wird das Verletzungs- und Infektionsrisiko im Umgang mit Blutentnahmegeräten verringert.

Im folgenden wir die Erfindung anhand eines Ausführungsbeispieles im Zusammenhang mit der Zeichnung näher erläutert:

Es zeigt:
- Fig. 1: einen Querschnitt durch eine Blutentnahmevorrichtung gemäß der Erfindung bei geschlossenen Hähnen;
- Fig. 2: eine Draufsicht auf die Blutentnahmevorrichtung;
- Fig. 3: einen vergrößerten Querschnitt durch das in Fig. 1 gezeigte Betätigungsorgan;
- Fig. 4: eine Ansicht des Betätigungsorganes von unten;
- Fig. 5: einen Querschnitt durch die Blutentnahmevorrichtung in Seitenansicht;
- Fig. 6: einen Längsschnitt durch den Entnahmeport;
- Fig. 7: eine perspektivische Darstellung des Abdeckorganes mit einem Hahn und einem Entnahmeport;
- Fig. 8: eine Draufsicht auf das Abdeckorgan in Schließstellung, wie sie in den Fig. 1 und 5 gezeigt ist;
- Fig. 9: eine Draufsicht auf das Abdeckorgan in einer Öffnungsstellung;
- Fig. 10a: eine schematische Darstellung der Blutentnahmevorrichtung in der in Fig. 2 gezeigten Stellungskombination "0";
- Fig. 10b: eine schematische Darstellung der Blutentnahmevorrichtung in einer Stellungskombination "1";
- Fig. 10c: eine schematische Darstellung der Blutentnahmevorrichtung in einer Stellungskombination "2";
- Fig. 10d: eine schematische Darstellung der Blutentnahmevorrichtung in einer Stellungskombination "3";
- Fig. 10e: eine schematische Darstellung der Blutentnahmevorrichtung in einer Stellungskombination "4".

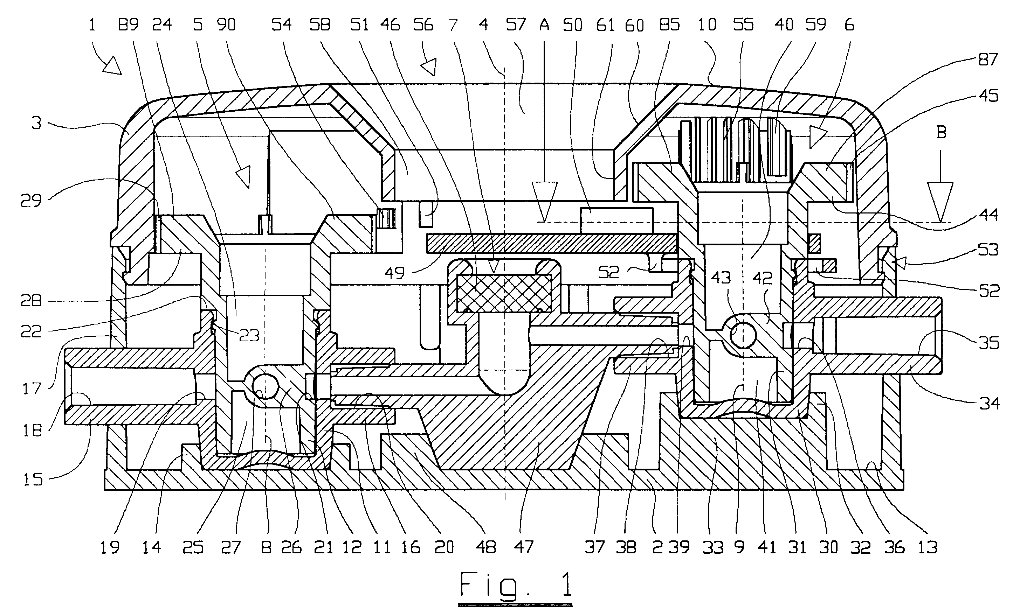

Fig. 1 zeigt eine Blutentnahmevorrichtung 1 mit einem Gehäuse 2 und einem Betätigungsorgan, das als deckelartiger Drehgriff 3 ausgeführt ist. Der deckelartige Drehgriff 3 ist um eine zentrale Achse 4 der Blutentnahmevorrichtung 1 gegenüber dem Gehäuse 2 drehbar.

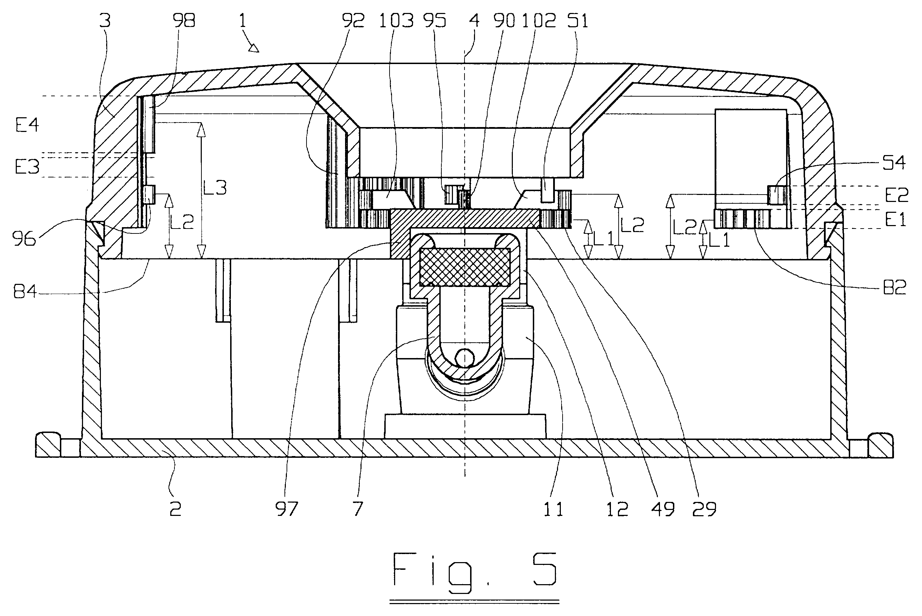

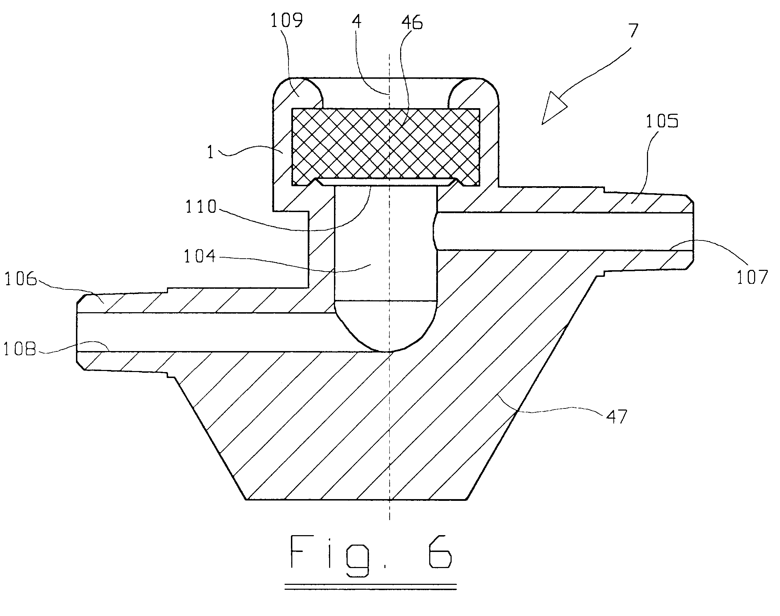

Im Gehäuse 2 der Blutentnahmevorrichtung 1 sind ein erster Hahn 5, ein zweiter Hahn 6 und ein Entnahmeport 7 angeordnet. Der Entnahmeport 7 ist zwischen den beiden Hähnen 5 und 6 angeordnet und mit diesen verbunden. Der erste Hahn 5 hat eine Drehachse 8 und der zweite Hahn 6 hat eine dazu parallele Drehachse 9. Der zweite Hahn 6 ist baugleich zum ersten Hahn 5, aber gegenüber diesem um 180° gedreht und zusätzlich axial versetzt im Gehäuse 2 angeordnet. Aufgrund des Versatzes des Hahns 6 entlang seiner Drehachse 9 und in Richtung zur Oberseite 10 des Drehgriffs 3 hin hat der Hahn 6 einen geringeren Abstand zum Drehgriff 3 als der Hahn 5. Somit liegen an den Küken 12 und 31 angebrachte Verzahnungen 29 bzw. 45 in 2 verschiedenen Ebenen, wodurch die Küken 12, 31 unabhängig voneinander verstellbar sind; dies wird im Zusammenhang mit den Figuren 3 und 5 noch näher erläutert. Ferner läßt sich durch den axialen Versatz der baugleichen Hähne 5 und 6 im daran angeschlossenen Entnahmeports 7 eine "Luftfalle" realisieren, wodurch sich die Bediensicherheit der Blutentnahmevorrichtung 1 zusätzlich erhöht; hierauf wird im Zusammenhang mit Fig. der 6 näher eingegangen.

Der Hahn 1 besteht aus einem Hahngehäuse 11 und einem darin angeordneten Küken 12. Das Hahngehäuse 11 sitzt unmittelbar auf dem Boden 13 des Gehäuses 2 auf. Ein seitliches Verrutschen des Hahnes 5 wird dadurch verhindert, daß das Hahngehäuse 11 teilweise in eine das Hahngehäuse 11 ringförmig umschließende Seitenhalterung 14 eingesetzt ist. Die Seitenhalterung 14 ist Teil des Gehäuses 2. Die Innenseite der Seitenhalterung 14 und der darin eingesetzte Teil des Hahngehäuses 11 sind konisch gestaltet, wodurch sich ein relativ fester Sitz des Hahngehäuses 11 im Gehäuse 2 ergibt. Das Hahngehäuse 11 weist einen Anschlußstutzen 15 und einen Verbindungsstutzen 16 auf. Der Anschlußstutzen 15 ist durch eine Gehäusewand 17 nach außen geführt. Ein Anschlußkanal 18 im Inneren des Anschlußstutzens 15 mündet über eine Öffnung 19 in den Innenraum des Hahngehäuses 11, in den das Küken 12 eingesetzt ist. Von der gegenüberliegenden Seite mündet ein im Verbindungsstutzen 16 vorhandener Verbindungskanal 20 über eine Öffnung 21 ebenfalls in den Innenraum des Hahngehäuses 11.

Das in das Hahngehäuse 11 eingesetzte Küken 12 ist über einen Absatz 22 gegenüber dem Hahngehäuse 11 axial abgestützt. Zur Abdichtung des Kükens 12 gegenüber dem Hahngehäuse 11 ist dieses an seiner Unterseite geschlossen und im oberen Bereich des Kükens 12 ist ein ringförmiger Dichtungswulst 23 vorgesehen, der den Schaft des Kükens 12 umschließt und eng an der Innenseite des Hahngehäuses 11 anliegt. Im oberen Bereich des Kükens 12 ist eine Ausnehmung 24 und im unteren Bereich eine Ausnehmung 25 vorgesehen. Durch diese Ausnehmungen 24, 25 läßt sich eine relativ gleichmäßige Wandstärke des Kükens 12 erreichen, was eine Herstellung des Kükens 12 als Gußteil (z. B. Herstellung aus Kunststoff und im Spritzgußverfahren) ermöglicht. Zwischen den beiden Ausnehmungen 24, 25 verbleibt ein Gußabschnitt 26, in dem ein das Küken mittig durchsetzender Kanal 27 vorgesehen ist. In der gezeigten Stellung des Kükens 12 relativ zum Hahngehäuse 11 steht dieser Kanal 27 senkrecht zum Anschlußkanal 18 und senkrecht zum Verbindungskanal 20 des Gehäuses, so daß der Hahn 5 geschlossen ist.

In seinem oberen Bereich ist das Küken 12 in Form eines Absatzes 28 radial nach außen erweitert. Am Absatz 28 ist eine Außenverzahnung 29 vorgesehen, die sich rings um den gesamten Absatz 28 erstreckt und somit das Küken 12 vollständig umschließt. Diese Außenverzahnung 29 dient zum Verdrehen des Kükens 12 gegenüber dem Hahngehäuse 11 um dessen Drehachse 8 und wirkt mit dem Drehgriff 3 zusammen.

Der mit dem Hahn 5 baugleiche und zu diesem versetzt angeordnete Hahn 6 besteht aus einem entsprechenden Hahngehäuse 30 und einem darin angeordneten Hahnküken 31. Das Hahngehäuse 30 ist in eine der Seitenhalterung 14 entsprechende Seitenhalterung 32 eingesetzt. Die Seitenhalterung 32 ist ein wangenartiger Fortsatz einer Erhebung 33 des Gehäuses 2. Auf dieser Erhebung 33 sitzt das Hahngehäuse 30 auf, wodurch sich der axiale Versatz zum Hahn 5 ergibt. Entsprechend dem Gehäuse 11 des Hahns 5 weist das Hahngehäuse 30 einen sich durch die Gehäusewand 17 erstreckenden Anschlußstutzen 34 mit einem Anschlußkanal 35 auf, der über eine Öffnung 36 in das Innere des Hahngehäuses 30 zum Küken 31 hin einmündet. Ein dem Verbindungsstutzen 16 entsprechender Verbindungsstutzen 37 liegt dem Anschlußstutzen 34 gegenüber und erstreckt sich vom Hahngehäuse 30 weg in Richtung zum Entnahmeport 7. Der Verbindungsstutzen 37 weist in seinem Inneren einen Verbindungskanal 38 auf, der über eine Öffnung 39 in das Innere des Hahngehäuses 30 zum Küken 31 hin einmündet.

Auch das Küken 31 weist eine obere Ausnehmung 40 und eine untere Ausnehmung 41 mit einem dazwischenliegenden Gußabschnitt 42 auf, der von einem dem Kanal 27 entsprechenden Kanal 43 durchsetzt ist. Der Kanal 43 steht senkrecht zum Verbindungskanal 38 und zum Anschlußkanal 35, so daß auch der Hahn 6 in der gezeigten Stellung geschlossen ist. In seinem oberen Bereich ist das Küken 31 durch einen dem Absatz 28 entsprechenden Absatz 44 radial nach außen erweitert und dadurch axial gegenüber dem Hahngehäuse 30 abgestützt. An der radialen Außenseite des Absatzes 44 weist das Küken 31 eine der Verzahnung 29 entsprechende Außenverzahnung 45 auf. Über diese Außenverzahnung 45 wird das Küken 31 mittels des Drehgriffs 3 gegenüber dem Hahngehäuse 30 verdreht und somit die Stellung des Hahnes 6 verändert.

Der zwischen den beiden Hähnen 5 und 6 angeordnete und mit diesen über die Verbindungsstutzen 16 bzw. 37 verbundene Entnahmeport 7 ist an seiner Oberseite durch einen Verschlußstopfen 46 verschlossen. In seinem unteren Bereich weist der Entnahmeport 7 einen plattenartig vom Entnahmeport 7 abstehenden Haltefortsatz 47 auf, der in eine Schlitzhalterung 48, die Teil des Gehäuses 2 ist, von oben her eingesetzt ist und somit den Entnahmeport 7 im Gehäuse hält. Eine detailliertere Erläuterung des Entnahmeports 7 erfolgt im Zusammenhang mit der Fig. 6.

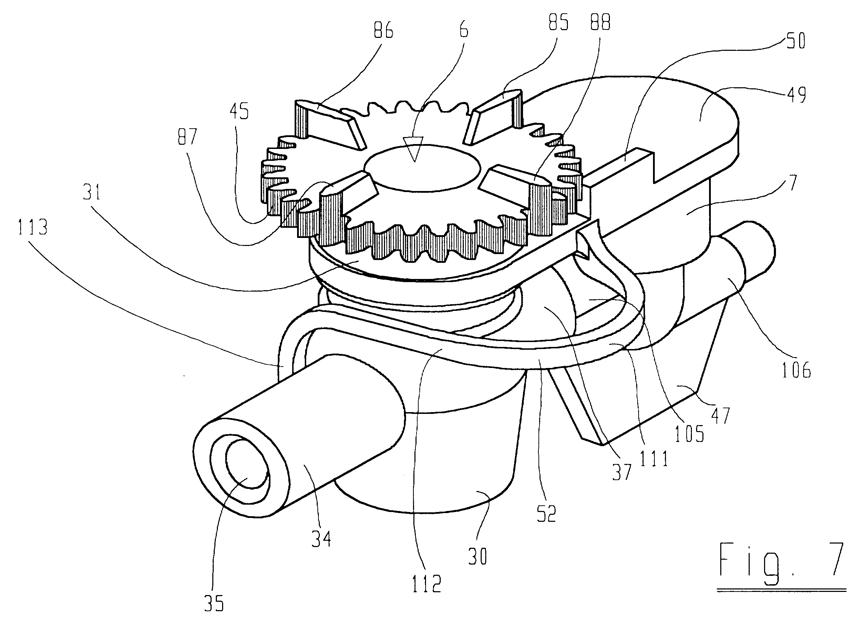

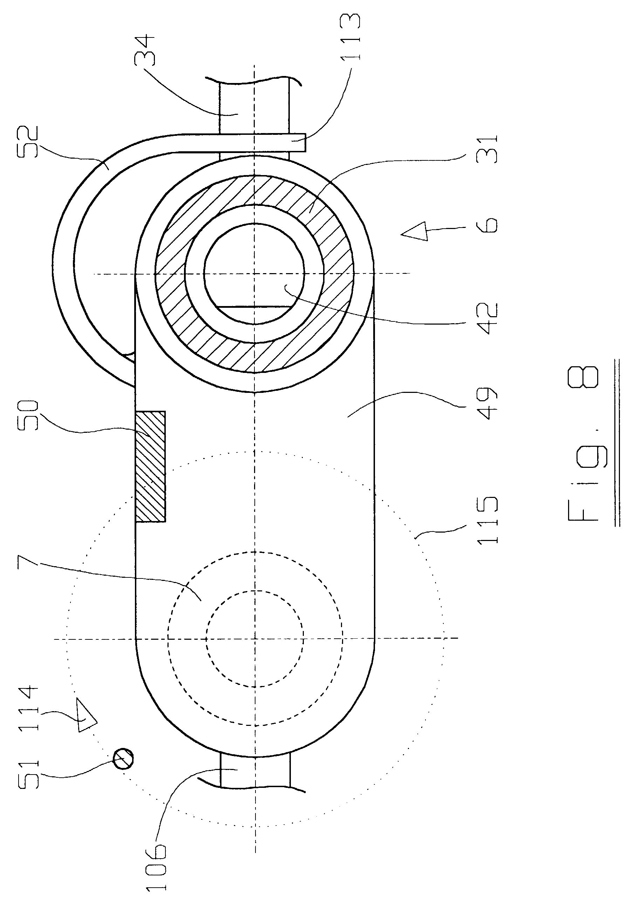

In der gezeigten Stellung des Drehgriffs 3 ist der Verschlußstopfen 46 des Entnahmeports 7 durch einen Abdeckarm 49 abgedeckt (vgl. Fig. 8). Der Abdeckarm 49 ist drehbar um die Drehachse 9 am Küken 31 des Hahnes 6 gelagert; das Küken 31 ist also relativ zum Abdeckarm 49 drehbar, so daß sich eine Drehung des Kükens 31 nicht auf den Abdeckarm 49 überträgt. An seiner Oberseite weist der Abdeckarm 49 ein Anschlagelement 50 auf, das bei Drehen des Drehgriffes 3 mit einem am Drehgriff 3 angeordneten Mitnehmerelement 51 zusammenwirkt. Die genaue Funktionsweise der Betätigung des Abdeckarms 49 wird im Zusammenhang mit den Fig. 7, 8 und 9 erläutert. Mit dem Abdeckarm 49 ist ein Federelement 52 verbunden, das im gezeigten Ausführungsbeispiel einstückig in den Abdeckarm 49 übergeht. Alternativ dazu kann das Federelement 52 auch ein separates Bauteil sein, das einseitig entweder mit dem Abdeckarm oder mit einem raumfesten Teil der Blutentnahmevorrichtung verbunden ist. In der gezeigten Darstellung ist das Federelement 52 hinter der Zeichenebene am Hahn 6 vorbeigeführt und am Anschlußstutzen 34 des Hahngehäuses 30 beweglich, d. h. geringfügig drehbeweglich abgestützt (vgl. Fig. 7, 8, 9). Alternativ dazu könnte das Federelement 49 auch fest mit dem Hahngehäuse 30 verbunden und am Abdeckarm 49 abgestützt sein.

Der die beiden Hähne 5 und 6 und den Abdeckarm 49 betätigende Drehgriff 3 ist über eine Rastverbindung 53 derart mit dem Gehäuse 2 verbunden, daß er um die zentrale Achse 4 relativ zum Gehäuse 3 drehbar aber axial dazu fest ist. Eine Betätigung der Hähne 5 und 6 und des Abdeckarms 49 erfolgt durch Drehen des Drehgriffes 3 um diese Achse 4.

Für die Verstellung der Hähne 5 und 6 sind an der Innenseite des Drehgriffs 3 einzelne Zähne und aus mehreren Zähnen bestehende Zahngruppen angeordnet, wovon zwei einzelne Zähne 54, 59 und eine Zahngruppe 55 zu sehen sind. Durch Verdrehen des Drehgriffes 3 greift der einzelne Zahn 54 ab einer bestimmten Stellung des Drehgriffes 3 in einen Teil (vgl. Fig. 3, 5 und dem analogen Küken 31, das in Fig. 7 gezeigt ist) der Außenverzahnung 29 des Kükens 12 des Hahns 5 ein und "nimmt das Küken mit", so daß dieses um einen relativ kleinen Winkel verdreht wird. Unter dem genannten "Teil" der Außenverzahnung 29 sind vier hervorstehenden Zähne 89, 90, 102, 103 des Kükens 12 zu verstehen. Durch das Mitnehmen eines der hervorstehenden Zähne 89, 90, 102, 103 wird sichergestellt, daß sich das Küken 12 in einer Drehstellung befindet, in der ein Eingreifen von zugeordneten Zahngruppen 82 (vgl. Fig. 5) des Drehgriffs 3 in die Außenverzahnung 29 des Kükens 12 möglich ist; Drehstellungen des Kükens 12, in denen es zu "Zahn auf Zahn-Stellungen" kommen könnte, werden durch die mit den hervorstehenden Zähnen 89, 90, 102, 103 zusammenwirkenden einzelnen Zähnen des Drehgriffs 3 (ordnungsgemäße Drehpositionierung des Kükens 12) sicher vermieden. Der dem Zahn 54 funktionell entsprechende einzelne Zahn 59 greift in hervorstehende Zähne 85, 86, 87, 88 des anderen Kükens 31 ein, die aus der übrigen Verzahnung 45 nach oben hervorstehen (vgl. Fig. 7); hierdurch wird auch das Küken 31 so positioniert, daß ein Zahneingriff in dessen Außenverzahnung 45 sicher möglich ist. Im Gegensatz zu dem Einzelzahn 59 erstreckt sich die Zahngruppe 55 weiter nach unten und kann somit mit allen Zähnen der Verzahnung 45 des Kükens 31 in Eingriff treten.

Nachdem ein einzelner Zahn 54, 59 bzw. eine Zahngruppe 55 in die entsprechende Außenverzahnung 29 bzw. 45 eingegriffen hat, wird durch Weiterdrehen des Drehgriffs 3 der zugehörige Hahn 5 bzw. 6 solange "mitgenommen", d.h. gegenüber dem Hahngehäuse 11 bzw. 30 verdreht, bis ein "letzter" Zahn die entsprechende Verzahnung 29 bzw. 55 verläßt. In der gezeigten Stellung des Drehgriffes 3 wird keines der beiden Hahnküken 12 bzw. 31 gedreht.

Zentral bezüglich der Achse 4 ist im Drehgriff 3 eine Öffnung 56 vorgesehen. In einem ersten Bereich 57 ist die zugehörige Wandung 60 der Öffnung 56 konisch und in einem zweiten Bereich 58 weist die Öffnung 56 eine zylindrische Wandung 61 auf. Die konische Form der ersten Wandung 60 erleichtert ein Einführen eines Blutentnahmegerätes (nicht dargestellt) und die zylindrische zweite Wandung 61 dient der Führung eines entsprechend gestalteten Blutentnahmegerätes, das bei geöffnetem Abdeckarm 49 von der Oberseite 10 des Drehgriffes 3 entlang der zentralen Achse 4 auf den Entnahmeport 7 aufgesetzt werden kann.

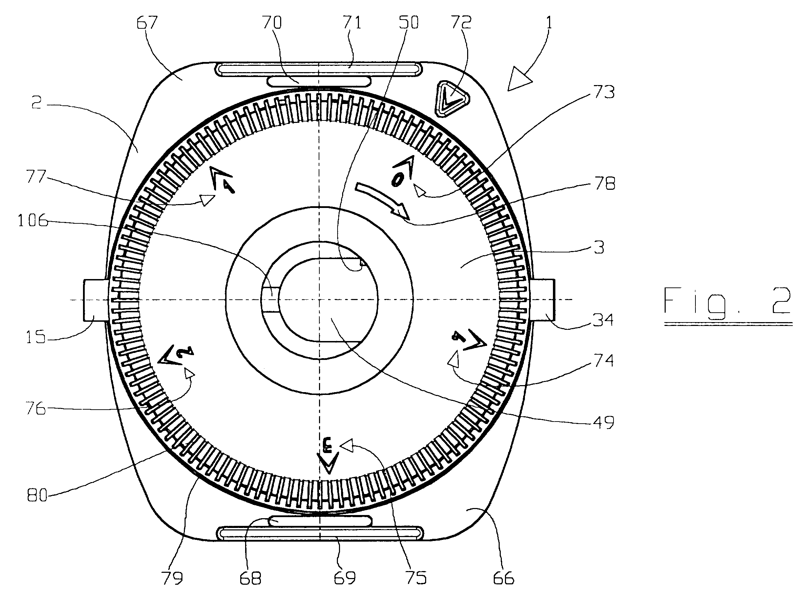

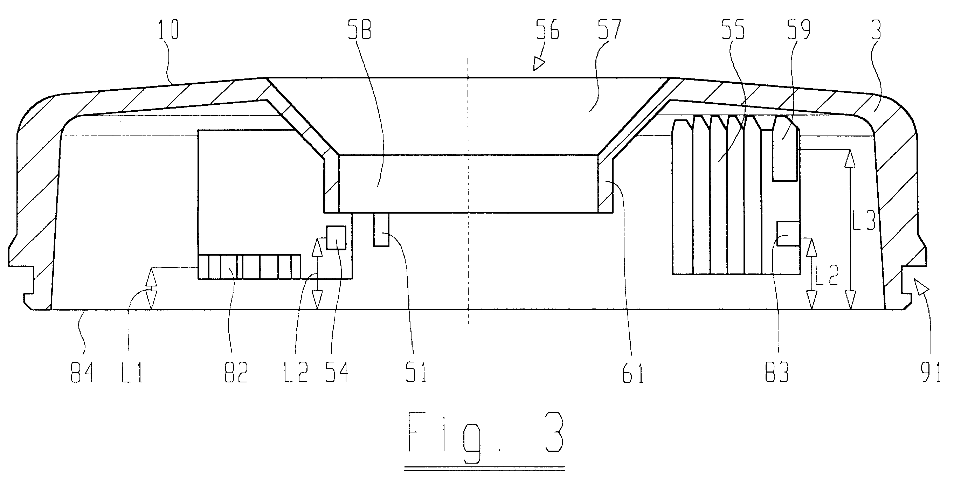

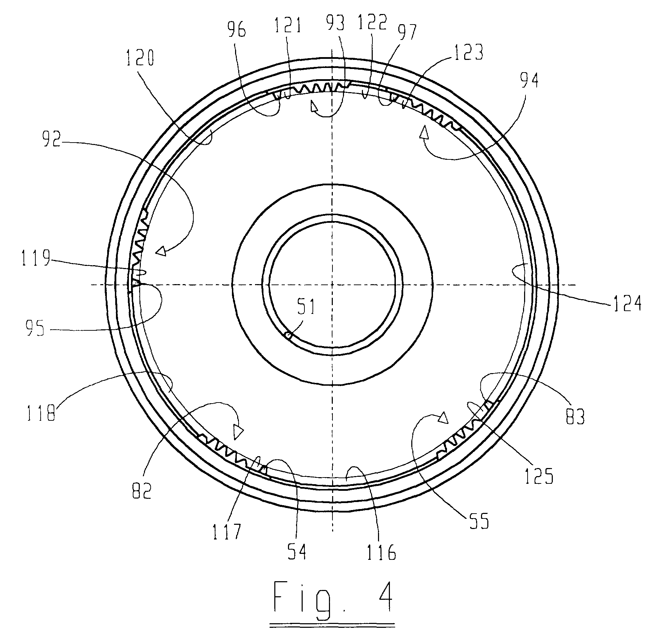

Fig. 2 zeigt eine Draufsicht auf die Blutentnahmevorrichtung 1 der Fig. 1. Das Gehäuse 2 ist an zwei gegenüberliegenden Enden 66 bzw. 67 plattenartig erweitert und weist an den Enden 66 und 67 je zwei Langlöcher 68, 69 bzw. 70, 71 auf. Mit diesen Langlöchern kann die Blutentnahmevorrichtung 1 durch Gurte beispielsweise am Arm eines Patienten befestigt werden.

Weiter ist zu erkennen, daß im Bereich des Endes 67 des Gehäuses 2 eine Bezugsmarkierung 73 angebracht ist. Ferner sind am Drehgriff Markierungen 73, 74, 75, 76, 77 angebracht. In der gezeigten Stellung des Drehgriffs liegt die Markierung 73 der Bezugsmarkierung 72 des Gehäuses gegenüber und zeigt somit eine Stellungskombination "0" der Blutentnahmevorrichtung 1 an. Ferner ist eine Pfeilmarkierung 78 am Drehgriff 3 vorgesehen, die anzeigt, in welche Richtung der Drehgriff 3 gedreht werden muß, um beispielsweise die bei einer Blutentnahme erforderlichen Stellungskombinationen in der richtigen Reihenfolge zu durchlaufen. Um ein Greifen bzw. Drehen des Drehgriffes 3 zu erleichtern, ist am gesamten Rand 79 des Drehgriffes 3 eine Riffelung 80 vorgesehen.

Durch die Öffnung 56 (vgl. Fig. 1) ist das Abdeckorgan 49, das Anschlagelement 50 und ein Verbindungsstutzen 106 (vgl. Fig. 6 und 7) des Entnahmeports 7 zu sehen. Ferner sind die aus dem Gehäuse 2 nach außen ragenden Anschlußstutzen 15 und 34 der Hähne 5 und 6 dargestellt.

Fig. 3 zeigt eine vergrößerte Darstellung des in Fig. 1 gezeigten Drehgriffes 3 der Blutentnahmevorrichtung 1. Deutlich ist in dieser Darstellung das Mitnehmerelement 51 zu erkennen, das eine "Fortsetzung" der zylindrischen Wandung 61 des zweiten Bereichs 58 der Öffnung 56 im Drehgriff 3 ist und somit in der gezeigten Stellung hinter der Zeichenebene liegt (vgl. Fig. 4 und 8).

Ferner sind die bereits in Fig. 1 gezeigten einzelnen Zähne 54, 59 und die Zahngruppe 55 zu erkennen; eine weitere zu erkennende Zahngrupppe 82 und ein weiterer einzelner Zahn 83 sind in Fig. 1 durch die Küken 12 bzw. 31 verdeckt. Die gezeigten einzelnen Zähne 54, 59, 83 und die Zahngruppen 55, 82 sind teilweise axial versetzt zueinander und haben entsprechend unterschiedliche Funktionen (vgl. Fig. 1, Fig. 7). Die Zahngruppe 82 hat einen mittleren Abstand L1 von einer Unterseite 84 des Drehgriffes 3 und liegt somit in einer Höhe über dem Gehäuse 2, in der alle Zähne der Außenverzahung 29 des Kükens 12 erfaßbar sind. Wie bereits erläutert, haben die Küken 31 bzw. 12 jeweils vier hervorstehende Zähne 85, 86, 87, 88 bzw. 89, 90, 102, 103 (vgl. Fig. 1, 5 und 7). Die hervorstehenden Zähne 89, 90, 102, 103 des Kükens 12 reichen bis in eine mittlere Höhe L2 über der Unterseite 84 des Drehgriffes 3 in Richtung zu dessen Oberseite 10 und werden somit außer durch die Zahngruppe 82 auch durch die beiden einzelnen Zähne 54 und 83 erfaßt. Die hervorstehenden Zähne 85, 86, 87, 88 des Kükens 31 reichen bis in eine mittlere Höhe L3 über der Unterseite 84 des Drehgriffes 3 nach oben und werden deshalb durch den einzelnen Zahn 59 erfaßt. Als Sonderfall ist die Zahngruppe 55 aufzufassen, die den gesamten Bereich der einzelnen Zähne 54, 59, 83 und der Zahngruppe 82 abdeckt und somit in alle Zähne der beiden Küken 12 und 31 eingreifen kann.

Ferner ist an der Außenseite des Drehgriffes 3 nahe der Unterseite 84 eine umlaufende Ausnehmung 91 zu erkennen, die einen Teil der in Fig. 1 gezeigten Rastverbindung 53 bildet.

Fig. 4 zeigt eine Unteransicht des in Fig. 3 gezeigten Drehgriffs 3. Außer den beiden Zahngruppen 55 und 82 und den einzelnen Zähnen 54 und 83 sind weitere Zahngruppen 92, 93, 94 und weitere einzelne Zähne 95, 96, 97 dargestellt, die, wie bereits erläutert ebenfalls teilweise axial versetzt zueinander sind. Zwischen den einzelnen Zähnen bzw. Zahngruppen sind zahnlose Lücken 116, 117, 118, 119, 120, 121, 122, 123, 124 und 125 vorgesehen.

Fig. 5 zeigt einen Querschnitt durch die Blutentnahmevorrichtung 1 mit dem Gehäuse 2 und dem Drehgriff 3 in Seitenansicht, in der das Hahngehäuse 11 und das Küken 12 zu sehen sind. Der Entnahmeport 7 ist, wie in Fig. 1 gezeigt, durch den Abdeckarm 49 überdeckt. Das in Fig. 1 gezeigte Anschlagelement 50 des Abdeckarmes 49 ist in dieser Darstellung nicht zu erkennen. Es ist aber ein weiteres Anschlagelement 97 zu erkennen, das in der gezeigten Stellung des Abdeckarmes 49 am Entnahmeport anliegt und das durch das Federelement 52 in dieser Stellung gehalten wird (vgl. Fig. 1, 7). Neben der bereits in Fig. 3 gezeigten Zahngruppe 82 und dem einzelnen Zahn 54 sind die Zahngruppe 92 (vgl. Fig. 3) und einzelne Zähne 95, 96, 98 zu erkennen. Diese einzelnen Zähne und Zahngruppen sind in den erläuterten unterschiedlichen mittleren Höhen L1, L2, L3 über der Unterseite 84 des Drehgriffes 3 angeordnet, was zusätzlich durch die "Drehebenen" E1, E2, E3 und E4 angedeutet ist. Die in der "Ebene" E1 liegende Zahngruppe 82 wirkt mit der in derselben Ebene liegenden Außenverzahnung 29 des Kükens 12 und die in der "Ebene" E2 liegenden hervorstehenden Zähne 102, 103 wirken mit den einzelnen Zähnen 54 und 96 zusammen. In der "Ebene" E3 liegende Zähne greifen in die Außenverzahnung 45 (vgl. Fig. 1) des Kükens 31 ein und in der Ebene E4 liegende Zähne greifen in dessen hervorstehende Zähne ein. Die zu erkennende Zahngruppe 92 erstreckt sich über alle vier Ebenen E1, E2, E3 und E4.

Neben dem bereits in Fig. 1 zu erkennenden hervorstehenden Zahn 90 des Kükens 12 sind zwei weitere hervorstehende Zähne 102, 103 des Kükens 12 zu erkennen.

Fig. 6 zeigt im Querschnitt den bereits in den Fig. 1 und 5 gezeigten Entnahmeport 7 im Detail. Der Entnahmeport 7 weist einen Hohlraum 104 auf, in den von gegenüberliegenden Seiten des Entnahmeports 7 in entsprechenden Verbindungsstutzen 105, 106 enthaltene Verbindungskanäle 107 bzw. 108 einmünden. Der Verbindungsstutzen 105 ist in den in Fig. 1 gezeigten Verbindungsstutzen 37 des Hahns 6 und der Verbindungsstutzen 106 ist in den Verbindungsstutzen 16 des Hahns 5 eingeschoben. Somit steht der Verbindungskanal 108 mit dem Hahn 5 und der Verbindungskanal 107 mit dem Hahn 6 in Verbindung. Der Kanal 107 ist parallel und axial, in Bezug auf die Achse 4, versetzt zum Kanal 108.

Im oberen Bereich ist der Entnahmeport 7 durch den Verschlußstopfen 46 verschlossen und weist eine umlaufende, wulstartige Erhebung 109 auf, die ein Ansetzen eines Blutentnahmegerätes (nicht dargestellt) erleichtert. Durch den Versatz der beiden Kanäle 107 und 108 ergibt sich eine sogenannte "Luftfalle"; unter der Annahme, daß die Schwerkraft vom Verschlußstopfen 46 zum Kanal 108, d. h. nach unten, gerichtet ist, schwimmen möglicherweise im Hohlraum enthaltene Luftblasen unmittelbar unterhalb der Unterseite 110 des Verschlußstopfens 46. Ist der Kanal 108 bzw. der damit verbundene Hahn 5 mit dem Patienten verbunden, so ergibt sich durch diese Luftfalle ein zusätzlicher Schutzeffekt, so daß sichergestellt ist, daß im Hohlraum 104 enthaltene Gasblasen nicht in den Kanal 108 bzw. zum Patienten gelangen können. Der Haltevortsatz 47 dient der Fixierung des Entnahmeports 7 im Gehäuse 2.

Fig. 7 zeigt eine perspektivische Ansicht des Hahnes 6 mit Hahngehäuse 30 und Küken 31, des Entnahmeportes 7 und des Abdeckorganes 49 mit dem Anschlagelement 50. Der Hahn 6 ist mit seinem Verbindungsstutzen 37 mit dem Verbindungsstutzen 105 des Entnahmeports 7, der dem Verbindungsstutzen 106 gegenüberliegt verbunden. In dieser Dartsellung sind besonders gut der Haltefortsatz 47 des Entnahmeports 7 und die hervorstehenden Zähne 85, 86, 87, 88 des Kükens 31 zu erkennen. Ferner ist das Federelement 52 zu erkennen, das sich auf einem Abschnitt 111 halbkreisförmig um den Hahn 6 erstreckt, das sich auf einem weiteren Abschnitt 112 weitgehend gerade über den Anschlußstutzen 35 erstreckt und das sich auf einem nach unten gekrümmten Abschnitt 113 in Richtung zum Boden 13 des Gehäuses 2 (vgl. Fig. 1) erstreckt und den Anschlußstutzen 34 teilweise umgreift. Beispielsweise ist das Federelement 52 in der gezeigten "Schließstellung" des Abdeckarmes 49 entspannt oder nur leicht vorgespannt.

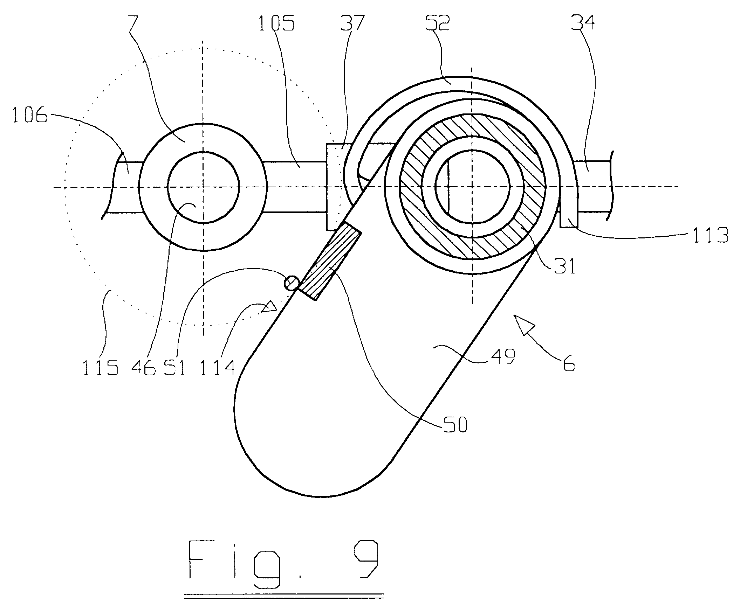

Fig. 8 zeigt einen Schnitt durch den Hahn 6 bzw. das Küken 31 und das Anschlagelement 50, entlang der in Fig. 1 angegebenen Schnittlinie A-B. Im Küken 31 ist ferner der Gußabschnitt 42 zu erkennen. Der Abdeckarm 49 befindet sich in Schließstellung, so daß der Entnahmeport 7 durch den Abdeckarm 49 verdeckt ist. Der Entnahmeport 7 ist deshalb gestrichelt eingezeichnet. Der mit dem Hahn 5 (nicht dargestellt) verbundene Verbindungsstutzen 106 und der Anschlußstutzen 113 sind in dieser Darstellung geschnitten. Durch Drehen des Drehgriffes 3 in Richtung des Pfeiles 114 wird das Mitnehmerelement 51 entlang der gepunkteten Kreisbahn 115 bewegt, bis es auf das Anschlagelement 50 auftritt und den Abdeckarm 49 auslenkt (vgl. Fig. 9).

Fig. 9 zeigt den am Hahn 6 gelagerten Abdeckarm 49 in einer Stellung nahe seiner maximalen Auslenkstellung, so daß der Entnahmeport 7 zugänglich ist. In dieser Stellung sind beide Verbindungsstutzen 105, 106 des Entnahmeports 7 und der Verbindungsstutzen des Hahns 6 zu erkennen. Durch Weiterdrehen des Drehgriffes 3 in Richtung des Pfeils 114 bewegt sich das Mitnehmerelement 51 auf der Kreisbahn 115, wodurch es den Anschlag 50 verläßt und der Abdeckarm 49 durch die aufgrund der Verformung des Federelementes 52 bewirkten Federkraft in die in Fig. 8 gezeigte Schließstellung zurückgeschwenkt wird.

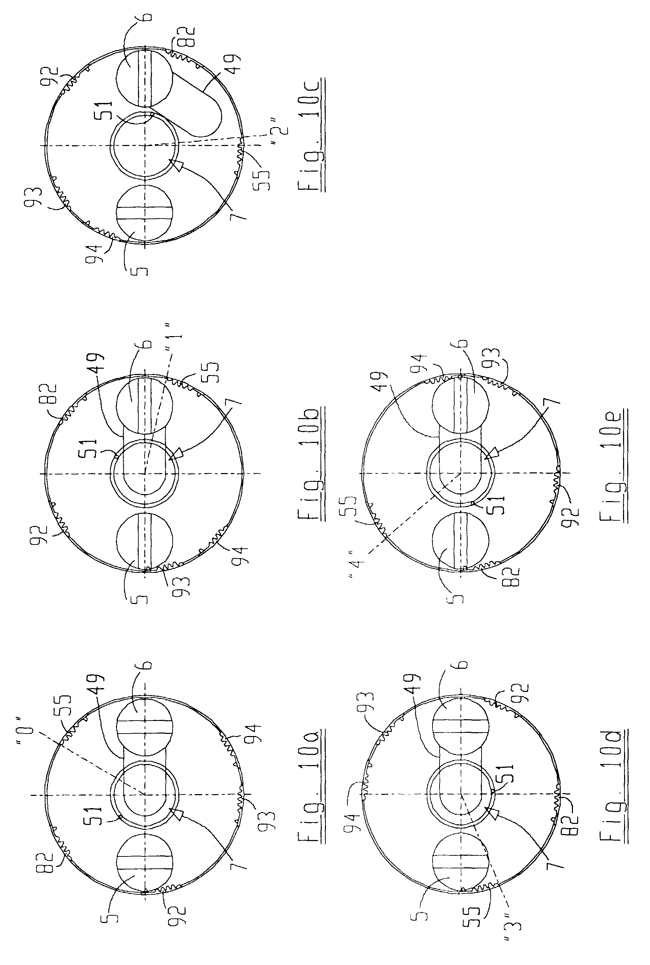

Die Fig. 10a, 10b, 10c, 10d und 10e zeigen schematisch fünf für eine Blutentnahme wichtige Stellungskombinationen der Blutentnahmevorrichtung entsprechend den Stellungen "0", "1", "2", "3" und "4" (vgl. Fig. 2) des Drehgriffs 3 (nicht dargestellt). Durch Betätigen des Drehgriffs 3 werden diese nacheinander durchlaufen.

In der in Fig. 10a gezeigten Stellung sind beide Hähne 5 und 6 und der Entnahmeport 7 durch das Abdeckorgan 49 geschlossen. Unter der Annahme, daß der Hahn 6 mit dem Patienten und der Hahn 5 mit einem Reservoir bzw. mit einer Infusionsflasche verbunden sind, kann weder Blut vom Patienten noch Infusionsflüssigkeit zum Entnahmeport 7 strömen und eine eventuell im Entnahmeport 7 enthaltene Flüssigkeit kann nicht entnommen werden. Durch Drehen des Drehgriffs 3 in Stellung "1" öffnet zunächst die Zahngruppe 92 den Hahn 5 und anschließend die Zahngruppe 55 den Hahn 6.

In dieser in Fig. 10b gezeigten Stellung "1" kann mit dem Reservoir Blut-Infusionsflüssigkeits-Gemisch angesaugt werden, bis der Entnahmeport mit reinem Blut gefüllt ist.

Durch Weiterdrehen des Drehgriffs 3 in Stellung "2" schließt die Zahngruppe 93 den Hahn 5. Da die Zahngruppe 82 in der "Ebene" des Hahns 5, nicht aber in der des Hahns 6 liegt, läuft diese am Hahn 6 vorbei, so daß dieser offen bleibt. Gleichzeitig bewirkt die Drehung des Drehgriffs 3, daß das Mitnehmerelement 51 den Abdeckarm 49 auslenkt und den Entnahmeport 7 (nicht dargestellt) freigibt. In dieser Stellung kann ein Blutentnahmegerät am Entnahmeport angesetzt werden und eine Blutennahme erfolgen.

Nach Abnehmen des Blutentnahmegeräts werden durch Weiterdrehen in Stellung "3" der Entnahmeport 7 durch den Abdeckarm 49 abgedeckt und der Hahn 6 durch die Zahngruppe 92 geschlossen. Der Hahn 5 wird hierbei nicht verstellt.

Durch Weiterdrehen in Stellung "4" öffnet die Zahngruppe 55 den Hahn 5 und die Zahngruppe 93 den Hahn 6, so daß durch das Reservoir abgesaugte Flüssigkeit dem Patienten wieder zugeführt werden kann.

Durch Weiterdrehen schließt die Zahngruppe 82 den Hahn 5 und die Zahngruppe 94 den Hahn 6, so daß man wieder in die Ausgangsstellung "0" gelangt.

## Patentansprüche

1. Blutentnahmevorrichtung mit einem Entnahmeport, der einen Hohlraum aufweist, in den zwei Kanäle und eine Öffnung münden, wobei die Öffnung durch einen mit einer Nadel durchdringbaren Verschluß verschlossen ist und mit jedem der beiden Kanäle des Entnahmeports je ein Hahn verbunden ist und die Hähne den zugeordneten Kanal absperren bzw. freigeben,
**dadurch gekennzeichnet,**
daß die Hähne (5, 6) und der Entnahmeport (7) raumfest zueinander angeordnet sind und ein bewegliches Abdeckorgan (49) vorgesehen ist, das in mindestens einer seiner möglichen Stellungen den Verschluß (46) des Entnahmeports (7) überdeckt und ihn in mindestens einer anderen Stellung freigibt und,
daß die Hähne (5, 6) mechanisch miteinander gekoppelt sind und
daß Stellungskombinationen der Hähne (5, 6) und des Abdeckorganes (49) durch die Stellung des gemeinsamen Betätigungsorganes (3) festgelegt sind.

2. Blutentnahmevorrichtung nach Anspruch 1,
**dadurch gekennzeichnet,**
daß die mechanische Kopplung zwischen dem Betätigungsorgan (3) und den Hähnen (5, 6) durch ineinandergreifende Zähne (29, 45, 54, 55, 59, 82, 83, 85, 86, 87, 88, 89, 90, 92, 93, 94, 95, 96, 97, 98, 102, 103) an den Hähnen (5, 6) und am Betätigungsorgan (3) gebildet ist.

3. Blutentnahmevorrichtung nach Anspruch 2,
**dadurch gekennzeichnet,**
daß die Zähne (54, 55, 59, 82, 83, 92, 93, 94, 95, 96, 97, 98) des Betätigungsorganes (3) in azimutaler Richtung abschnittsweise mit Lücken (116, 117, 118, 119, 120, 121, 122, 123, 124, 125) angeordnet sind, in denen die Zähne (29, 45, 85, 86, 87, 88, 89, 90, 102, 103) an den Hähnen (5, 6) außer Eingriff sind.

4. Blutentnahmevorrichtung nach einem der Ansprüche 2 oder 3,
**dadurch gekennzeichnet,**
daß die Zähne (29, 89, 90, 102, 103) des einen Hahnes (5) gegenüber den Zähnen (45, 85, 86, 87, 88) des anderen Hahnes (6) axial versetzt sind.

5. Blutentnahmevorrichtung nach einem der Ansprüche 2 bis 4,
**dadurch gekennzeichnet,**
daß an den beiden Hähnen (5, 6) einige Zähne (85, 86, 87, 88, 89, 90, 102, 103) vorgesehen sind, die breiter als die anderen Zähne des zugeordneten Hahns (5, 6) sind und daß am Betätigungsorgan (3) Zähne (54, 59, 83, 95, 96) vorhanden sind, die ausschließlich in die verbreiterten Zähne (85, 86, 87, 88, 89, 90, 102, 103) des zugeordneten Hahnes (5, 6) eingreifen.

6. Blutentnahmevorrichtung nach einem der Ansprüche 2 bis 5,
**dadurch gekennzeichnet,**
daß an den Hähnen (5, 6) jeweils eine Außenverzahnung (29, 45, 85, 86, 87, 88, 89, 90, 102, 103) und am Betätigungsorgan (3) eine Innenverzahnung (54, 55, 59, 82, 83, 92, 93, 94, 95, 96, 97, 98) vorgesehen ist.

7. Blutentnahmevorrichtung nach einem der Ansprüche 1 bis 6,
**dadurch gekennzeichnet,**
daß die Kopplung zwischen dem Abdeckorgan (49) und dem Betätigungsorgan (3) durch ein Mitnehmerelement (51) am Betätigungsorgan (3) und ein Anschlagelement (50) am Abdeckorgan (49) gebildet ist und das Abdeckorgan (49) von einer Stellung in eine andere entgegen der Kraft eines Federelementes (52) bewegt wird.

8. Blutentnahmevorrichtung nach einem der Ansprüche 1 bis 7,
**dadurch gekennzeichnet,**
daß das Abdeckorgan ein Abdeckarm (49) ist, der an einem der beiden Hähne (5, 6) schwenkbar gelagert ist.

9. Blutentnahmevorrichtung nach einem der Ansprüche 1 bis 6,
**dadurch gekennzeichnet,**
daß das Abdeckorgan eine drehbare Abdeckscheibe mit einer Durchgangsöffnung ist.

10. Blutentnahmevorrichtung nach einem der Ansprüche 1 bis 9,
**dadurch gekennzeichnet,**
daß das Betätigungsorgan (3) ein deckelartiger Drehgriff (3) ist.

11. Blutentnahmevorrichtung nach Anspruch 10,
**dadurch gekennzeichnet,**
daß der Drehgriff (3) beide Hähne (5, 6) und den Entnahmeport (7) überdeckt und im Bereich über dem Verschluß (46) des Entnahmeports (7) eine Öffnung (56) mit einer Führungsfläche (60, 61) für ein einzuführendes Blutentnahmegerät aufweist.

12. Blutentnahmevorrichtung nach einem der Ansprüche 1 bis 11,
**dadurch gekennzeichnet,**
daß der Entnahmeport (7) und die beiden Hähne (5, 6) in einem gemeinsamen Gehäuse (2) angeordnet sind.
